# EUROPÄISCHE PATENTANMELDUNG

(11) **EP 1 752 125 A1**
(43) Veröffentlichungstag der Anmeldung: **14.02.2007**
(21) Anmeldenummer: 05017585.0
(22) Anmeldetag: 12.08.2005
(51) Int. Cl.: A61H 35/00, A61N 1/44

(54) **Elektrolysefussbadgerät und Konverterspuleneinheit**

(71) Anmelder: Body Detox AG, 8400 Winterthur (CH)
(72) Erfinder: Mosimann, François, 8400 Winterthur (CH); Artmann, Heribert, 93455 Traitsching - Höhhof (DE)
(74) Vertreter: Koepe, Gerd L.

(57) **Zusammenfassung**

Eine Konverterspuleneinheit für ein Elektrolysefußbadgerät weist ein integriertes Anschlussmittel auf. Das Elektrolysefußbadgerät für die Konverterspuleneinheit weist eine Andockeinrichtung auf, an die die Konverterspuleneinheit mittels des Anschlussmittels andockbar ist.

## Beschreibung

Die Erfindung betrifft ein Elektrolysefußbadgerät und eine Konverterspuleneinheit, die mit dem Elektrolysefußbadgerät betreibbar ist.

In der Medizin, in der Heilkunde und im Wellness-Bereich werden Elektrolysefußbäder als therapeutische Maßnahme zur Entschlackung und Entgiftung des menschlichen Körpers verordnet. Hierbei werden über die Haut der in eine Solelösung eingetauchten Füße toxische oder belastende aber auch körpereigene Stoffe ausgeschieden. Insbesondere werden bei der Anwendung des Elektrolysefußbads Schwermetalle, Harnsäure, Calcium, Magnesium, chlorierte Kohlenwasserstoffe, Biphenyle, usw. aus den Körper ausgeleitet.

Zur Durchführung eines Elektrolysefußbads werden herkömmliche Elektrolysefußbadgeräte verwendet, die eine Wanne und eine Konverterspule aufweisen. Die Wanne ist mit eventuell mit Zusätzen versehenem Wasser gefüllt, in das die Konverterspule eingetaucht ist. Die Konverterspule weist zwei Elektroden auf, die zueinander ein elektrisches Potential haben, aufgrund dessen eine Ionisierung des Wassers erzeugt wird. Sobald ein Patient seine Füße in das Wasser eintaucht, tritt über die von der Konverterspule erzeugte Ionisierung des Wassers die Wirkung des Elektrolysefußbads ein.

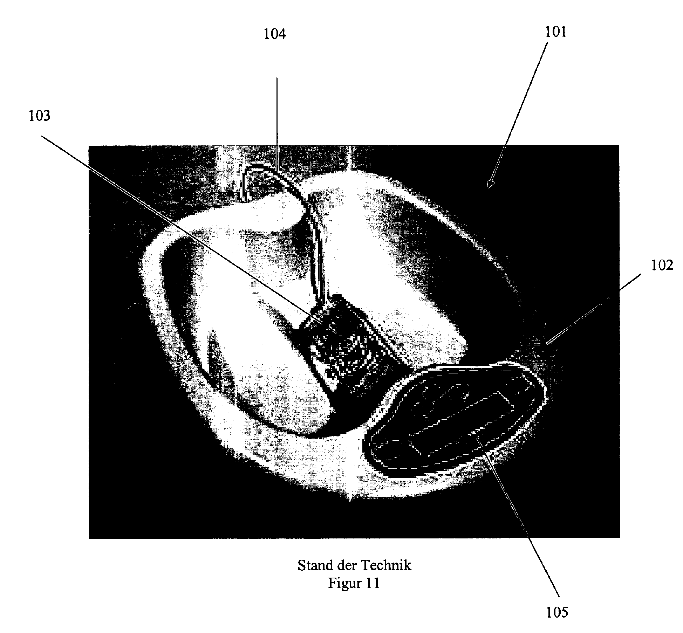

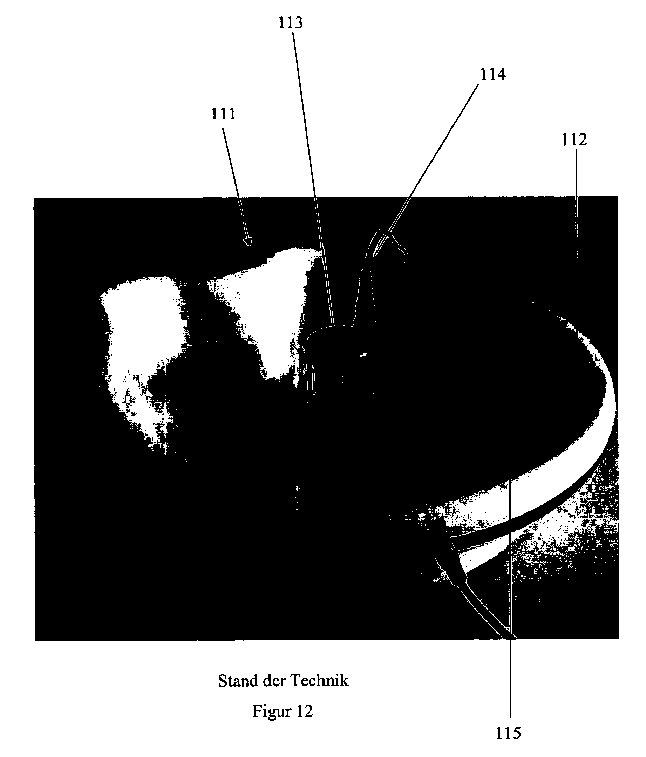

Herkömmliche Elektrolysefußbadgeräte mit entsprechenden herkömmlichen Konverterspulen sind in Figuren 11 und 12 gezeigt. Diese herkömmlichen Elektrolysefußbadgeräte 101, 111 weisen eine Wanne 102, 112 auf, in die die Fußbadflüssigkeit unterbringbar ist. Die herkömmliche Konverterspule 103, 113 weist zwei schraubenwendelförmige zueinander konzentrisch angeordnete Elektroden auf, die in einer offenen Gehäusekonstruktion untergebracht sind. Die Elektroden sind mittels eines Kabels 104, 114 mit einem Betriebsgerät 105, 115 zum Betrieb der Konverterspule 103, 113 verbunden. Die Konverterspule 103, 113 ist auf den Boden der Wanne 102, 112 gelegt oder gestellt, wobei die Konverterspule 103, 113 in der mit Fußbadflüssigkeit gefüllten Wanne 102, 112 untergetaucht ist.

Dadurch, dass das Kabel 104, 114 der Konverterspule 103, 113 in der Wanne 102, 112 verlegt ist, besteht die Gefahr, dass ein Patient, der ein Elektrolysefußbad nimmt, an dem Kabel 104, 114 hängen bleibt und sich daran verletzt oder dass die Konverterspule 103, 113 in der Wanne 102, 112 unerwünscht verrutscht. Ferner kann die Isolierung des Kabels 104, 114 brechen oder verschleißen, wodurch Wasser in das Kabel 104, 114 eindringen kann, wodurch eine unerwünschte elektrische Kontaktierung des Wassers mit dem Betriebsgerät 105, 115 resultieren kann. Dies kann beispielsweise zu einem Kurzschluss im Betriebsgerät 105, 115 führen oder dazu, dass der Patient einen Stromstoss beim Fußbaden erleidet. Außerdem besteht die Gefahr, dass das Kabel 104, 114 innerhalb der Isolierung bricht und somit einen Wackelkontakt aufweist. Dies führt zu einer Betriebsstörung des Elektrolysefußbadgeräts 101, 111.

Aufgabe der Erfindung ist es, ein Elektrolysefußbadgerät und eine Konverterspuleneinheit dafür zu schaffen, wobei das Elektrolysefußbadgerät und die Konverterspuleneinheit sicher im Betrieb und einfach in der Handhabung sind.

Die erfindungsgemäße Konverterspuleneinheit für ein Elektrolysefußbadgerät weist ein integriertes Anschlussmittel und das erfindungsgemäße Elektrolysefußbadgerät für die Konverterspuleneinheit weist eine Andockeinrichtung auf, an die die Konverterspuleneinheit mittels des Anschlussmittels andockbar ist. Die Konverterspuleneinheit bildet mit ihrem Anschlussmittel eine integrale Einheit, wodurch die Konverterspuleneinheit an die Andockeinrichtung direkt anschließbar ist. Dadurch sind keine außerhalb der Konverterspuleneinheit oder außerhalb des Elektrolysefußbadgeräts angesiedelten zusätzlichen Anschlussmittel notwendig, wie beispielsweise zusätzlich zwischen der Konverterspuleneinheit und dem Elektrolysefußbadgerät vorgesehene Kabel, wodurch das Anschließen der Konverterspuleneinheit an das Elektrolysefußbadgerät einfach bewerkstelligt werden kann. Dadurch ist die Handhabbarkeit des Elektrolysefußbadgeräts und der Konverterspuleneinheit verbessert und die elektrische Verbindung zwischen der Konverterspuleneinheit und dem Elektrolysefußbadgerät zuverlässig und sicher.

Der Oberflächenabschnitt der Konverterspuleneinheit, der mit dem Gegenoberflächenabschnitt der Andockeinrichtung in Kontakt steht, kann beispielsweise Stiftvorsprünge aufweisen, die in entsprechende Löcher in dem Gegenoberflächenabschnitt eingreifen. Bevorzugt weist das Anschlussmittel einen Oberflächenabschnitt, der stumpf an die Andockeinrichtung zur Herstellung eines elektrischen Kontakts an dem Oberflächenabschnitt anlegbar ist, und die Andockeinrichtung einen Gegenoberflächenabschnitt auf, an den stumpf das Anschlussmittel zur Herstellung eines elektrischen Kontakts an dem Gegenoberflächenabschnitt anlegbar ist. Dadurch, dass die beiden Oberflächenabschnitte, nämlich der Oberflächenabschnitt des Anschlussmittels und der Gegenoberflächenabschnitt der Andockeinrichtung, stumpf ausgebildet sind, können diese beispielsweise durch Abwischen mit einem Tuch oder einem Schwamm einfach gereinigt werden.

Beispielsweise können der Oberflächenabschnitt und der Gegenoberflächenabschnitt plan ausgeführt sein. Bevorzugt weist der Oberflächenabschnitt Vertiefungen auf, wobei der Gegenoberflächenabschnitt plan ausgeführt ist. Zwischen den Vertiefungen des Oberflächenabschnitts bilden sich von diesem vorstehende Abschnitte aus, an denen der Gegenoberflächenabschnitt anlegbar ist. Ist der Oberflächenabschnitt an den Gegenoberflächenabschnitt angelegt, bilden sich die Vertiefungen als Hohlräume aus, die von den vorstehenden Abschnitten abgegrenzt sind. Ist der Oberflächenabschnitt und/oder der Gegenoberflächenabschnitt vor dem Zusammenfügen der beiden Oberflächenabschnitte beispielsweise mit Wasser benetzt, so besteht die Gefahr, dass ein durchgehender Wasserfilm sich zwischen den Oberflächenabschnitten ausbildet, wenn die Oberflächenabschnitte aneinandergelegt werden. Dieser Wasserfilm würde einerseits einer stabilen mechanischen Verbindung zwischen dem Anschlussmittel und der Andockeinrichtung entgegenstehen und andererseits zu einem elektrischen Kurzschluss an dem Anschlussmittel und/oder der Andockeinrichtung führen.

Abhilfe schaffen hier die erfindungsgemäßen Vertiefungen in dem Oberflächenabschnitt, da der Wasserfilm von den vorstehenden Abschnitten des Oberflächenabschnitts unterbrochen wird und die vorstehenden Abschnitte des Oberflächenabschnitts den Gegenoberflächenabschnitt berühren, wobei sich von den vorstehenden Abschnitten des Oberflächenabschnitts verdrängtes Wasser in den Hohlräumen ansammelt. Dadurch kann sich zwischen dem Oberflächenabschnitt und dem Gegenoberflächenabschnitt, wenn das Anschlussmittel an die Andockeinrichtung angelegt ist, kein durchgehender Wasserfilm ausbilden, wodurch die mechanische Stabilität der Verbindung zwischen dem Anschlussmittel und der Andockeinrichtung hoch ist und die Gefahr eines elektrischen Kurzschlusses der elektronischen Verbindung zwischen dem Anschlussmittel und der Andockeinrichtung unterbunden ist.

Bevorzugt weist das Anschlussmittel wenigstens einen in das Anschlussmittel eingelassenen und an dem Oberflächenabschnitt von außen zugänglichen Kontaktstift, und die Andockeinrichtung wenigstens einen in die Andockeinrichtung eingelassenen und an dem Gegenoberflächenabschnitt von außen zugänglichem Gegenkontaktstift auf, wobei der Kontaktstift mit dem Gegenkontaktstift kontaktierbar ist. Somit ist der elektrische Kontakt zwischen dem Anschlussmittel und der Andockeinrichtung mittels des Kontaktstifts und des Gegenkontaktstifts zuverlässig und einfach herstellbar.

Bevorzugt steht der Kontaktstift von dem Oberflächenabschnitt vor und bevorzugt ist der Gegenkontaktstift in der Andockeinrichtung versenkt. Liegt das Anschlussmittel mit dem Oberflächenabschnitt an dem Gegenoberflächenabschnitt der Andockeinrichtung an, so ragt der Kontaktstift in die Andockeinrichtung hinein, wobei der Kontaktstift den Gegenkontaktstift berührt. Da somit der Kontaktstift in der Andockeinrichtung seitlich gehalten ist, wird ein seitliches Verrutschen des Kontaktstifts gegenüber dem Gegenkontaktstift verhindert, so dass die elektrische Verbindung zwischen dem Kontaktstift und dem Gegenkontaktstift unempfindlich gegen äußere mechanische Einflüsse ist.

Bevorzugt ist der Kontaktstift in dem Anschlussmittel längsverschiebbar und der Gegenkontaktstift als Senkniet ausgeführt, wobei der Kontaktstift mit dem Gegenkontaktstift in Eingriff bringbar ist. Der Senkniet ist einfach an der Andockeinrichtung anbringbar, wodurch die Herstellung des Elektrolysefußbadgeräts kostengünstig ist. Da der Kontaktstift im Anschlussmittel längsverschiebbar ist, ist er mit dem Senkniet, dessen Kopf im Wesentlichen plan mit der Oberfläche des Gegenoberflächenabschnitts ist, einfach in Kontakt zu bringen, indem der Kontaktstift mit seiner Längsachse im Wesentlichen mit der Längsachse des Senkniets fluchtend angeordnet ist und so weit längsverschoben wird, bis er an den Senkniet anstößt. Dadurch ist ein zuverlässiger und einfacher elektrischer Kontakt zwischen dem Kontaktstift und dem Gegenkontaktstift herstellbar.

Bevorzugt weist das dem Oberflächenabschnitt zugewandte Ende des Kontaktstifts eine konvexe Struktur und das dem Gegenoberflächenabschnitt zugewandte Ende des Gegenkontaktstifts eine konkave Struktur auf, wobei die Strukturen zueinandergehörig entsprechend geformt sind, so dass sie formschlüssig zusammenwirken können. Bei der Kontaktbildung zwischen dem Kontaktstift und dem Gegenkontaktstift stehen beide Stifte miteinander formschlüssig in Eingriff, wodurch aufgrund der konvexen bzw. konkaven Ausbildung der Strukturen die Stifte eine Zentrierungswirkung bezüglich einer gemeinsamen Längsachse erfahren. Dadurch ist vorteilhaft der elektrische Kontakt großflächig ausgebildet und gegen versehentliches seitliches Verschieben von einem der Stifte stabil.

Bevorzugt weist das Anschlussmittel ein Vorspannmittel auf, das den Kontaktstift in Richtung aus dem Anschlussmittel heraus vorspannt. Dadurch wird auf den Kontaktstift von dem Vorspannmittel eine Vorspannkraft ausgeübt, die bei angedocktem Anschlussmittel auf den Gegenkontaktstift einwirkt. Dadurch wird vorteilhaft erreicht, dass der Kontaktstift gegen den Gegenkontaktstift gedrückt wird, wodurch ein guter elektrischer Kontakt zwischen den Stiften erzielt ist.

Bevorzugt ist das Vorspannmittel eine Druckfeder. Die Druckfeder ist einfach in der Herstellung und einfach zu montieren. Dadurch ist vorteilhaft das Elektrolysefußbadgerät kostengünstig in der Herstellung.

Bevorzugt weisen sowohl das Anschlussmittel als auch die Andockeinrichtung ein Haltemittel zum Halten des an die Andockeinrichtung angedockten Anschlussmittels in Position bezogen auf die Andockeinrichtung auf. Dadurch ist mittels der Haltemittel die Position des Anschlussmittels bezogen auf die Andockeinrichtung festgelegt. Somit ist die Konverterspuleneinheit an dem Elektrolysefußbadgerät befestigt, wodurch vorteilhaft ein sicherer und zuverlässiger Betrieb des Elektrolysefußbadgeräts mit der Konverterspuleneinheit erzielt ist. Ferner ist der elektrische Kontakt zwischen der Konverterspuleneinheit und dem Elektrolysefußbadgerät vorteilhaft zuverlässig gegen Verrutschen.

Bevorzugt sind die Haltemittel Magnete, die jeweils an dem Oberflächenabschnitt in das Anschlussmittel und an dem Gegenoberflächenabschnitt in die Andockeinrichtung eingelassen sind. Die Magnete wirken durch magnetische Kraftwirkung zusammen, so dass das Anschlussmittel mit dem Oberflächenabschnitt an dem Gegenoberflächenabschnitt der Andockeinrichtung anhaftet. Dadurch, dass hierbei der Oberflächenabschnitt an dem Gegenoberflächenabschnitt anliegt und die beiden Oberflächenabschnitte durch die magnetische Kraftwirkung zwischen den Magneten aneinander gedrückt werden, ist das Anschlussmittel an der Andockeinrichtung festgehalten, wobei die beiden Oberflächenabschnitte flüssigkeitsdicht aneinander liegen. Somit ist der Kontaktstift und der Gegenkontaktstift flüssigkeitsdicht von der Umgebung abgeschlossen, wodurch vorteilhaft durch Eindringen von Flüssigkeit zwischen den Oberflächenabschnitten ein Kurzschluss unterbunden wird.

Die Magnete können Elektromagnete sein. Bevorzugt sind die Magnete Permanentmagnete. Permanentmagnete haben ein dauerhaftes Magnetfeld von sich aus, wodurch erreicht wird, dass keinerlei zusätzliche Betriebsmittel für die Haltemittel notwendig sind und die Handhabung der Konverterspuleneinheit beim Andocken an die Andockeinrichtung mit dem Anschlussmittel einfach zu handhaben ist. Ferner sind Permanentmagnete kostengünstig in der Anschaffung.

Die Konverterspuleneinheit weist bevorzugt eine Konverterspule und das Anschlussmittel eine Verbindungseinrichtung auf, mittels der via den Kontaktstift die Konverterspule mit der Andockeinrichtung in elektrische Verbindung bringbar ist. Dadurch ist die Konverterspuleneinheit modular aufgebaut, so dass die Konverterspule, die Verbindungseinrichtung und der Kontaktstift in separaten Herstellungsschritten herstellbar sind, wodurch vorteilhaft die Herstellung der Konverterspuleneinheit einfach und kostengünstig ist.

Bevorzugt ist die Verbindungseinrichtung eine eine Logik aufweisende Leiterplatte, an die der Kontaktstift angebaut und die Konverterspule angeschlossen ist, und bevorzugt weist die Andockeinrichtung eine eine Logik aufweisende Gegenleiterplatte auf, an der der Gegenkontaktstift angebaut ist. Dadurch, dass sowohl in dem Anschlussmittel als auch in der Andockeinrichtung logische Bauteile vorgesehen sind, kann der Betrieb der Konverterspule und/oder die Herstellung des elektrischen Kontakts zwischen der Konverterspuleneinheit und dem Elektrolysefußbadgerät mittels dieser logischen Bauteile gesteuert werden. Dadurch ist vorteilhaft ein optimierter Betrieb des Elektrolysefußbadgeräts mit der Konverterspule ermöglicht.

Die Verbindungseinrichtung weist bevorzugt ein Steckverbinderteil auf, mittels dessen die Konverterspule an die Leiterplatte angeschlossen ist. Dadurch ist ein modularer Aufbau der Konverterspuleneinheit ermöglicht, so dass diese in separaten Herstellungsschritten herstellbar ist, wodurch vorteilhaft die Herstellung der Konverterspuleneinheit einfach und kostengünstig ist.

Bevorzugt weist die Konverterspuleneinheit einen Trägerkörper auf, von dem die Konverterspule getragen und in dem das Anschlussmittel integriert ist. Dadurch weist vorteilhaft die Konverterspule eine kompakte Bauform auf, was günstig hinsichtlich der Platzverhältnisse in dem Elektrolysefußbadgerät ist.

Der Trägerkörper weist bevorzugt einen Hantelkörper auf, der von einer Stange und jeweils an deren Enden angeordnete Teller gebildet wird. Ferner weist bevorzugt die Konverterspule einen Windungsabschnitt und einen Zuleitungsabschnitt auf. Der Windungsabschnitt ist um den Hantelabschnitt von diesem getragen angeordnet, wobei die Stange im Innern des Windungsabschnitts sich erstreckt und die Teller jeweils an den Stirnseiten des Windungsabschnitts angeordnet sind. Dadurch ist der Außenumfang des Windungsabschnitts nicht von dem Trägerkörper umgeben, wodurch der Trägerkörper von außen frei zugänglich ist. Dadurch kann vorteilhaft der Windungsabschnitt von außen manuell mechanisch von an ihm abgelagerten Verschmutzungen gereinigt werden. Da der Windungsabschnitt nicht von beispielsweise Verstrebungen des Trägerkörpers umgeben ist, ist der Windungsabschnitt beim Betrieb des Elektrolysefußbadgeräts vollständig und ungehindert von Wasser umspült, wodurch eine Ionisierung des Wassers optimal erreicht wird.

Bevorzugt weist der Trägerkörper ferner einen hohlen Kanalabschnitt auf, der an einem der Teller beginnend von diesem sich weg erstreckt, wobei der Zuleitungsabschnitt innerhalb des Kanalabschnitts angeordnet ist. Ferner ist bevorzugt das Anschlussmittel am freien Ende des Kanalabschnitts angesiedelt, so dass die Konverterspule an dem Zuleitungsabschnitt mit der Andockeinrichtung in eine elektrische Verbindung bringbar ist. Somit ist sowohl der Zuleitungsabschnitt als auch das Anschlussmittel geschützt in dem Kanalabschnitt untergebracht, wodurch der Zuleitungsabschnitt und das Anschlussmittel gegenüber äußeren Einflüssen unempfindlich ist, wie beispielsweise mechanische Stöße oder korrosive Einwirkungen. Dadurch hat die Konverterspuleneinheit eine hohe Robustheit. Ist die Konverterspuleneinheit mit ihrem Anschlussmittel an der Andockeinrichtung befestigt, so wirkt der Kanalabschnitt als Haltestiel für den Hantelabschnitt zum Halten desselben in Position, wobei der Windungsabschnitt an dem Hantelabschnitt abgestützt ist. Somit wird der Windungsabschnitt via den Hantelabschnitt und via den Kanalabschnitt von dem Anschlussmittel an der Andockeinrichtung gehalten, wodurch die Konverterspuleneinheit vorteilhaft vollständig festgelegt ist und eine weitere Abstützung der Konverterspuleneinheit nicht vorgesehen zu werden braucht.

Bevorzugt ist der Kanalabschnitt L-förmig geformt, wobei der eine Schenkel radial nach außen von dem Hantelabschnitt aus gesehen geführt ist und der andere Schenkel axial von dem Hantelabschnitt aus gesehen von der Stange sich weg erstreckt. Da einerseits das Anschlussmittel am freien Ende des Kanalabschnitts angesiedelt ist und andererseits der Kanalabschnitt L-förmig geformt ist, besteht vorteilhaft die Möglichkeit, die elektrische Verbindung zur Konverterspuleneinheit axial und radial entfernt von dem Ort des Windungsabschnitts anzusiedeln, wenn dieser Ort beispielsweise zur Herstellung der elektrischen Verbindung ungeeignet ist, beispielsweise wenn der Windungsabschnitt beim Betrieb des Elektrolysefußbadgeräts untergetaucht ist.

Bevorzugt weist das Elektrolysefußbadgerät eine Fußbadschalenanordnung auf, in die die Andockeinrichtung integriert ist. Ist die Konverterspuleneinheit in der Fußbadschalenanordnung installiert, so kann die Konverterspuleneinheit direkt an die Fußbadschalenanordnung via die Andockeinrichtung angeschlossen werden. Somit kann vorteilhaft die Konverterspuleneinheit in eine gewünschte Position in der Fußbadschalenanordnung verbracht direkt an die Fußbadschalenanordnung, beispielsweise kabellos, angeschlossen werden.

Der Oberflächenabschnitt ist bevorzugt an der der Längsachse des Hantelabschnitts zugewandten Seite des außenliegenden Schenkels des Kanalabschnitts angesiedelt und der Gegenoberflächenabschnitt ist bevorzugt als Teil der Oberfläche der Fußbadschalenanordnung an einem Ort angesiedelt, der oberhalb der Normfüllhöhe der Fußbadschalenanordnung ist. Dadurch ist es vorteilhaft ermöglicht, dass die Stelle, an der die elektrische Verbindung zwischen der Konverterspuleneinheit und dem Elektrolysefußbadgerät hergestellt wird, außerhalb des in die Fußbadschalenanordnung in Normfüllhöhe eingefüllten Wassers angesiedelt ist. Dadurch ist diese elektrische Verbindungsstelle dauerhafter Feuchtigkeit nicht ausgesetzt. Somit wird vorteilhaft die Gefahr eines Kurzschlusses an dieser elektrischen Verbindungsstelle vermindert.

Außerdem ist es ermöglicht unter Ausnutzung dieses Vorteils die Konverterspule liegend in der Fußbadschalenanordnung in angeschlossenem Zustand vollständig untergetaucht anzuordnen, wodurch die Normfüllhöhe der Fußbadschalenanordnung als niedrig angesiedelt werden kann. Hierbei ist vorteilhaft nur eine geringe Menge an Wasser in der Fußbadschalenanordnung notwendig, wobei eine optimale Ionisationswirkung der Konverterspuleneinheit erzielt ist.

Ferner ist an der dem Oberflächenabschnitt abgewandten Seite des Kanalabschnitts eine Öse als Griff angebracht. Somit steht die Öse radial nach außen vor von dem Hantelabschnitt aus gesehen, wodurch, wenn die Konverterspuleneinheit an der Fußbadschalenanordnung angeschlossen ist, die Konverterspuleneinheit zum Montieren/Demontieren oder Herausnehmen aus der Fußbadschalenanordnung von oben her gut greifbar ist.

Bevorzugt ist der Gegenoberflächenabschnitt außerhalb der Fußbadschalenanordnung angesiedelt. Dadurch ist es vorteilhaft ermöglicht, dass die Andockeinrichtung beispielsweise in eine von der Fußbadschalenanordnung separate Vorrichtung integriert und entsprechend verkabelt ist. Dadurch braucht die Fußbadschalenanordnung nicht mit elektronischen Einrichtungen zum Betrieb der Konverterspuleneinheit zu versehen sein, wodurch die Fußbadschalenanordnung einfach und kostengünstig herstellbar ist. Ferner, da die zur Versorgung der Andockeinrichtung notwendige Elektronik außerhalb der Fußbadschalenanordnung untergebracht ist, ist die Gefahr gering, dass beim Hantieren des Elektrolysefußbadgeräts Wasser versehentlich an die Elektronik gerät. Dadurch ist vorteilhaft die Gefahr vermindert, dass in dieser Elektronik ein Kurzschluss auftritt.

Bevorzugt ist der Trägerkörper einstückig geformt und bevorzugt aus spritzgussfähigem Kunststoff hergestellt. Dadurch kann vorteilhaft der Trägerkörper kostengünstig in großen Stückzahlen hergestellt werden.

Bevorzugt sind die erste und/oder die letzte Windung des Windungsabschnitts in dem jeweils benachbarten Teller vergossen oder alternativ an Vorsprüngen an dem jeweils benachbarten Teller festgelegt, so dass die Konverterspule auf dem Hantelabschnitt gehalten ist. Dadurch wird einerseits eine kostengünstig herstellbare und feste Fixierung des Windungsabschnitts an dem Trägerkörper erreicht und andererseits vorteilhaft erzielt, dass durch diese Befestigung die einzelnen Windungen des Windungsabschnitts bei während des Betriebs des Elektrolysefußbadgeräts üblich auftretenden mechanischen Einwirkungen sich nicht berühren. Dadurch wird verhindert, dass der Windungsabschnitt im Betrieb der Konverterspuleneinheit sich versehentlich kurzschließt.

Bevorzugt sind an dem Elektrolysefußbadgerät zwei Kontakte zur Energieübertragung und zwei Kontakte zur Datenübertragung vorgesehen. Der Gegenoberflächenabschnitt weist bevorzugt eine erhabene Stelle auf, die mit einer dazugehörigen zurückversetzten Stelle an dem Oberflächenabschnitt formschlüssig zusammenwirken kann. Dadurch ist ein seitliches Verrutschen der Konverterspuleneinheit an dem Oberflächenabschnitt bezogen auf den Gegenoberflächenabschnitt unterbunden, wodurch vorteilhaft eine hohe Stabilität gegen seitliches Verrutschen der Konverterspuleneinheit bezogen auf die Andockeinrichtung erzielt ist.

Im Folgenden wird die Erfindung anhand bevorzugter Ausführungsformen mit Bezugnahme auf die Zeichnung erläutert.
Figur 1 ist eine perspektivische Darstellung einer Ausführungsform eines erfindungsgemäßen Elektrolysefußbadgeräts, in dem eine Ausführungsform einer erfindungsgemäßen Konverterspuleneinheit installiert ist,
Figur 2 ist die Draufsicht des Elektrolysefußbadgeräts aus Figur 1 und der Konverterspuleneinheit aus Figur 1,
Figur 3 ist eine Seitenansicht des Elektrolysefußbadgeräts und der Konverterspuleneinheit,
Figur 4 ist eine perspektivische Ansicht des Elektrolysefußbadgeräts,
Figur 5 ist eine perspektivische Ansicht der Konverterspuleneinheit,
Figur 6 ist eine Draufsicht der Konverterspuleneinheit,
Figur 7 ist eine Seitenansicht der Konverterspuleneinheit,
Figur 8 ist eine Explosionsdarstellung der Konverterspuleneinheit,
Figur 9 ist eine Längsschnittdarstellung durch das Elektrolysefußbadgerät und der Konverterspuleneinheit, die in dem Elektrolysefußbadgerät installiert ist,
Figur 10 ist eine vergrößerte Detaildarstellung des rechten Abschnitts aus Figur 9 und
Figuren 11 und 12 sind herkömmliche Elektrolysefußbadgeräte.

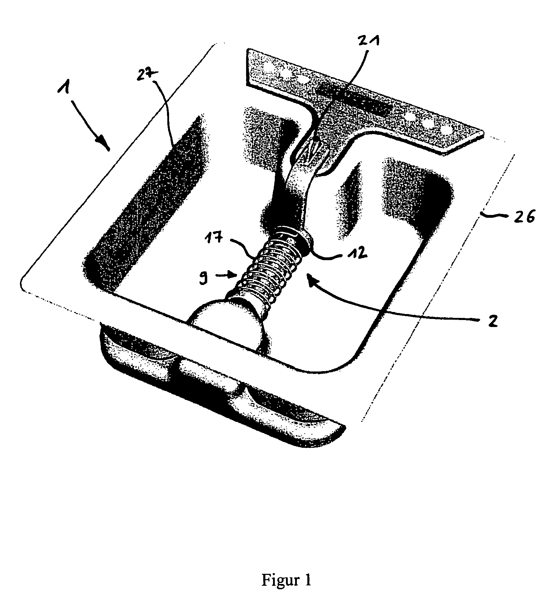

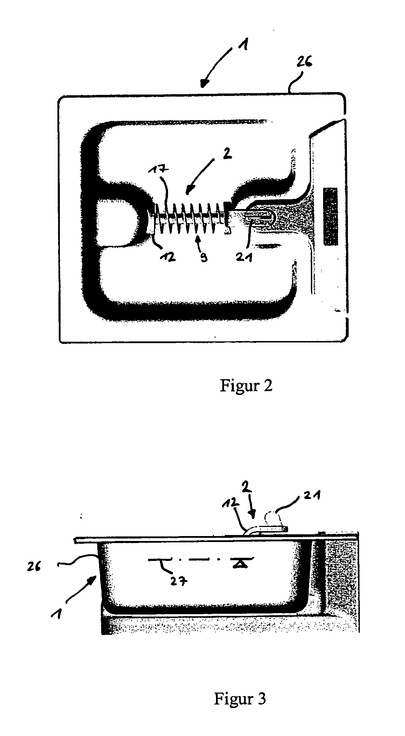

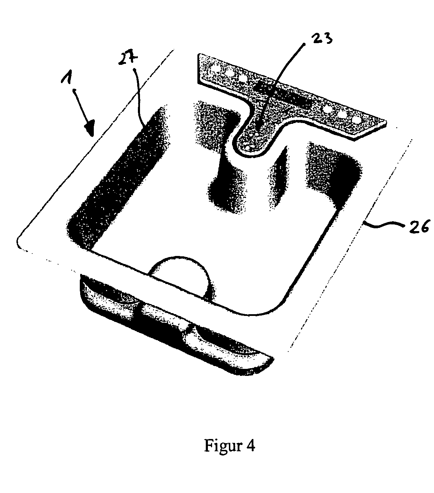

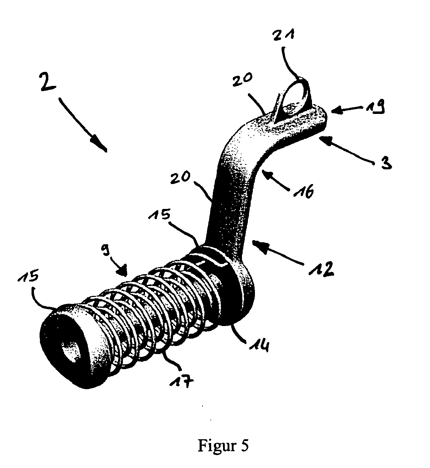

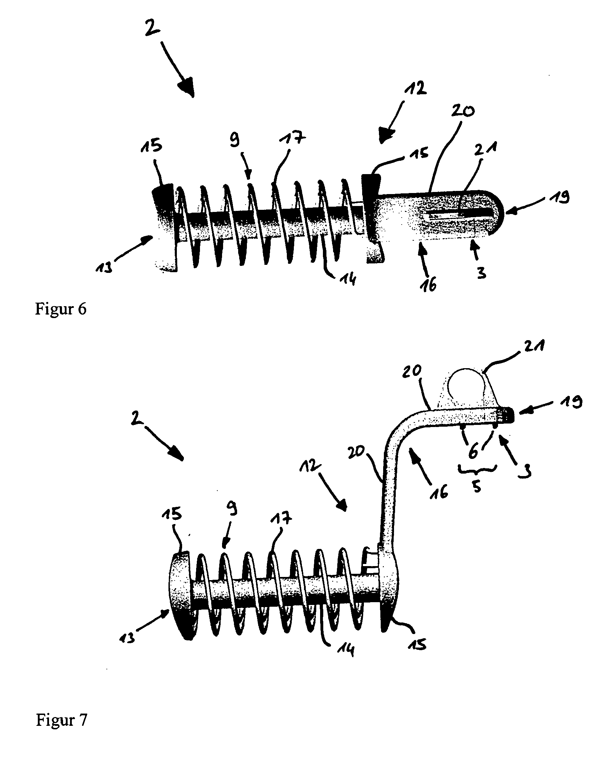

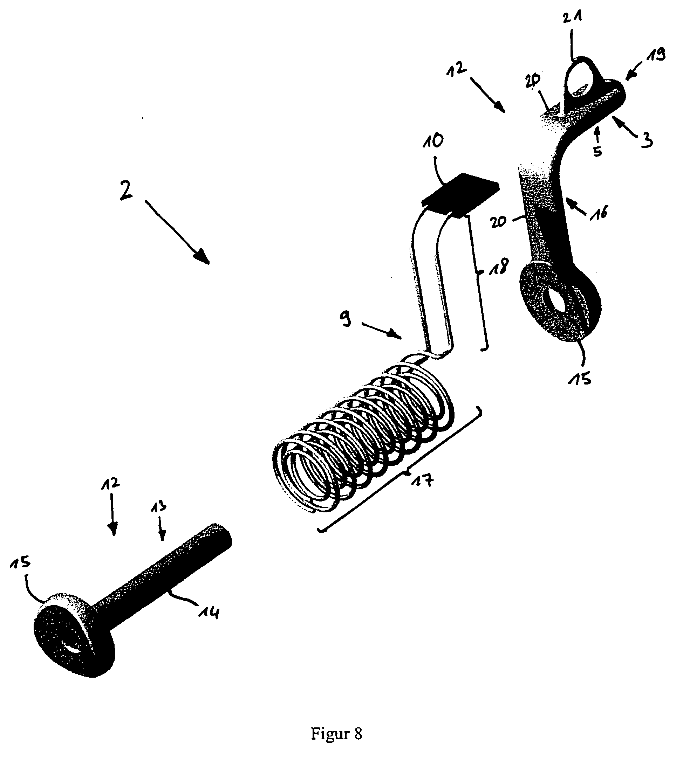

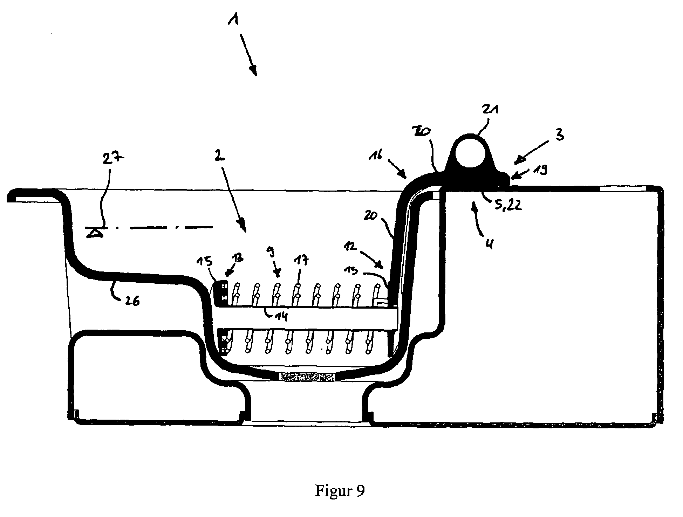

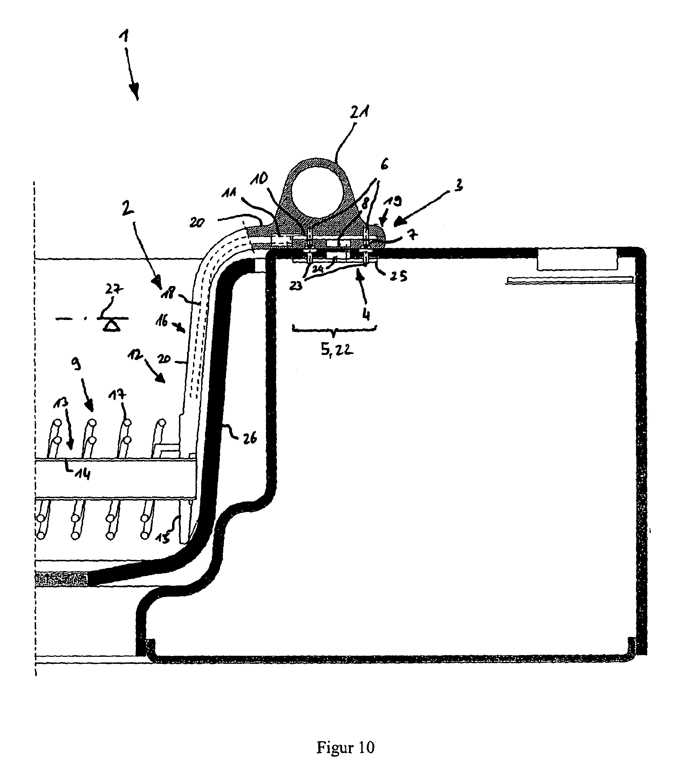

Wie aus den Figuren 1 bis 10 ersichtlich ist, ist in einem erfindungsgemäßen Elektrolysefußbadgerät 1 eine Konverterspuleneinheit 2 eingebaut. Die Konverterspuleneinheit 2 weist ein Anschlussmittel 3 und das Elektrolysefußbadgerät 1 weist eine Andockeinrichtung 4 auf, wobei das Anschlussmittel 3 in der Konverterspuleneinheit 2 integriert ist und die Konverterspuleneinheit 2 mittels des Anschlussmittels 3 an die Andockeinrichtung 4 angedockt ist.

Die Konverterspuleneinheit 2 weist eine Konverterspule 9 und einen Trägerkörper 12 auf, der die Konverterspule 9 abstützt. Die Konverterspule 9 weist einen Windungsabschnitt 17 auf, der von zwei schraubenwindungsförmigen, zueinander konzentrisch angeordneten Drahtelektroden gebildet ist. Ferner weist die Konverterspule 9 einen Zuleitungsabschnitt 18 auf, der als Verlängerung der Drahtelektroden des Windungsabschnitts 17 ausgebildet ist. Die Konverterspule 9 weist ferner einen Trägerkörper 12 auf, der von einer Stange 14 und jeweils an deren Enden angeordnete scheibenförmige Tellern 15 gebildet wird.

Der Windungsabschnitt 17 ist so um den Hantelabschnitt 13 angeordnet, dass die Stange 14 im Inneren des Windungsabschnitts 17 sich erstreckt und die Teller 15 jeweils an den Stirnseiten des Windungsabschnitts 17 angeordnet sind. Der Trägerkörper 12 ist aus Kunststoff hergestellt, so dass die Stirnseiten des Windungsabschnitts 17 in den jeweils benachbarten Teller vergossen sind. Dadurch ist der Windungsabschnitt 17 von dem Trägerkörper 12 stabil gehalten. Ferner, da der Windungsabschnitt 17 von außen frei zugänglich ist, kann dieser von außen manuell mechanisch gereinigt werden, beispielsweise wenn sich an dem Windungsabschnitt 17 durch den Betrieb Verschmutzungen abgelagert haben. Ferner wird im Betrieb der Windungsabschnitt 17 von in das Elektrolysefußbadgerät gefülltem Wasser vollständig und ungehindert umspült, wodurch eine optimale Ionisierung des Wassers erreicht wird.

Die Konverterspuleneinheit 2 weist einen Kanalabschnitt 16 auf, wobei der Kanalabschnitt 16 an demjenigen Teller 15 vorgesehen ist, an dem der Zuleitungsabschnitt 18 der Konverterspule 9 angesiedelt ist. Der Kanalabschnitt 16 ist L-förmig ausgebildet und ist mit seinem einen Schenkel 20 in Verlängerung zu dem Teller 15 radial nach außen geführt. Außen an diesen Schenkel 20 anschließend ist der andere Schenkel 20 des Kanalabschnitts 16 angeordnet, wobei dort der Kanalabschnitt 16 parallel zur Axialrichtung des Trägerkörpers 12 sich von diesem weg erstreckt. An dem freien Ende 19 des Kanalabschnitts 16 ist das Anschlussmittel 3 vorgesehen.

Das Elektrolysefußbadgerät 1 weist eine Fußbadschalenanordnung 26 auf, in die die Konverterspuleneinheit 2 eingebaut ist. Dabei ist die Konverterspule 9 in der Fußbadschalenanordnung 26 knapp oberhalb deren Boden liegend angeordnet, so dass unter Befüllung der Fußbadschalenanordnung 26 bis zu deren Normfüllhöhe 27 mit Wasser der Windungsabschnitt 17 vollständig untergetaucht ist.

Die Andockeinrichtung 4 ist in die Fußbadschalenanordnung 26 integriert, wobei die Andockeinrichtung 4 an dem oberen Rand der Fußbadschalenanordnung 26 angeordnet ist. Somit ist die Andockeinrichtung 4 oberhalb der Normfüllhöhe 27 der Fußbadschalenanordnung 26 angebracht, wodurch sowohl die Andockeinrichtung 4 und das Anschlussmittel 3 als auch die elektrische Verbindung zwischen der Andockeinrichtung 4 und dem Anschlussmittel 3 dem dauerhaften Untergetauchtsein in dem Wasser nicht ausgesetzt sind und dafür nicht ausgelegt zu sein brauchen. Dies führt bei der Herstellung des Elektrolysefußbadgeräts 1 und der Konverterspuleneinheit 2 zu einem Kostenvorteil und zu einer erhöhten Sicherheit im Betrieb, da ein Kurzschluss an der Verbindung zwischen dem Anschlussmittel 3 und der Andockeinrichtung 4 unterbunden ist.

Die Länge des radialen Schenkels 20 des Kanalabschnitts 16 ist derart dimensioniert, dass dieser Schenkel 20 vom äußeren Rand des Tellers 15 bis im Wesentlichen zur Oberkante der Fußbadschalenanordnung 26 sich erstreckt. Die Länge des anderen Schenkels 20 ist so dimensioniert, dass dieser vom äußeren Ende des radialen Schenkels 20 bis zur Andockeinrichtung 4 hin axial geführt ist. An dem freien Ende 19 des Kanalabschnitts 16 ist das Anschlussmittel 3 an der der Längsachse des Hantelabschnitts 13 zugewandten Seite des axialen Schenkels 20 des Kanalabschnitts 16 angebracht, so dass das Anschlussmittel 3 an die Andockeinrichtung 4 angedockt ist, wobei der Windungsabschnitt 17 der Konverterspule 9 in das in die Fußbadschalenanordnung 26 gefüllte Wasser liegend angeordnet untergetaucht ist.

An der dem Anschlussmittel 3 abgewandten Seite des axialen Schenkels 20 des Kanalabschnitts 16 ist eine nach oben vorstehende Öse 21 angebracht. Die Öse 21 kann von oben her gut gegriffen werden, so dass unter Lösen der Verbindung zwischen dem Anschlussmittel 3 und Andockeinrichtung 4 die Konverterspuleneinheit 2 bequem und sicher aus dem Elektrolysefußbadgerät 1 gehoben oder in das Elektrolysefußbadgerät 1 eingesetzt werden kann.

Das Anschlussmittel 3 weist einen Oberflächenabschnitt 5 auf und die Andockeinrichtung 4 weist einen dem Oberflächenabschnitt 5 entsprechenden Gegenoberflächenabschnitt 22 auf. Der Oberflächenabschnitt 5 ist im Wesentlichen bündig mit der Oberfläche des Kanalabschnitts 16 ausgebildet und der Gegenoberflächenabschnitt 22 ist im Wesentlichen bündig mit der Oberfläche der Fußbadschalenanordnung 26 ausgebildet, so dass das Anschlussmittel 3 an der Andockeinrichtung 4 stumpf anliegt. Dadurch stehen sowohl an der Andockeinrichtung 4 als auch an dem Anschlussmittel 3 keine Vorsprünge vor, wodurch sich sowohl das Anschlussmittel 3 als auch die Andockeinrichtung 4 leicht reinigen lassen und die Gefahr vermindert ist, dass ein Patient sich an dem Anschlussmittel 3 oder an der Andockeinrichtung 4 verletzt.

Der Gegenoberflächenabschnitt 22 ist plan ausgebildet, wobei der Oberflächenabschnitt 5 wellig ausgebildet ist. Dadurch liegt der Oberflächenabschnitt 5 an seinen vorstehenden Abschnitten an dem Gegenoberflächenabschnitt 22 an, wodurch zwischen den vorstehenden Abschnitten des Oberflächenabschnitts 5 sich Hohlräume ausbilden. Ist der Oberflächenabschnitt 5 und/oder der Gegenoberflächenabschnitt 22 vor dem Zusammenfügen der beiden Oberflächenabschnitte 5, 22 beim Herstellen der Verbindung zwischen dem Anschlussmittel 3 und der Andockeinrichtung 4 mit Wasser benetzt, beispielsweise aufgrund von Bespritzen mit Wasser, so besteht die Gefahr, dass ein durchgehender Wasserfilm zwischen dem Oberflächenabschnitt 5 und dem Gegenoberflächenabschnitt 22 sich ausbildet, wenn die beiden Oberflächenabschnitte 5, 22 zusammengefügt werden. Dieser durchgehende Wasserfilm kann einerseits einer stabilen mechanischen Verbindung zwischen dem Anschlussmittel 3 und der Andockeinrichtung 4 entgegenstehen und kann andererseits zu einem elektrischen Kurzschluss an dem Anschlussmittel 3 und/oder der Andockeinrichtung 4 führen. Ist das Anschlussmittel 3 mit seinem Oberflächenabschnitt 5 auf den Gegenoberflächenabschnitt 22 der Andockeinrichtung 4 gelegt, so berühren die vorstehenden Abschnitte des Oberflächenabschnitts 5 die plane Oberfläche des Gegenoberflächenabschnitts 22, wobei zwischen den vorstehenden Abschnitten des Oberflächenabschnitts 5 sich Hohlräume ausbilden. Dadurch wird ein eventuell auf dem Gegenoberflächenabschnitt 22 sich befindlicher durchgehender Wasserfilm von den vorstehenden Abschnitten des Gegenoberflächenabschnitts 22 unterbrochen, wobei sich von den vorstehenden Abschnitten des Oberflächenabschnitts 5 verdrängtes Wasser in den Hohlräumen ansammelt. Dadurch kann sich zwischen dem Oberflächenabschnitt 5 und dem Gegenoberflächenabschnitt 22, wenn das Anschlussmittel 3 an die Andockeinrichtung 4 angelegt ist, kein durchgehender Wasserfilm ausbilden, wodurch die mechanische Stabilität der Verbindung zwischen dem Anschlussmittel 3 und der Andockeinrichtung 4 hoch ist und die Gefahr eines elektrischen Kurzschlusses der elektronischen Verbindung zwischen dem Anschlussmittel 3 und der Andockeinrichtung 4 unterbunden ist.

An dem freien Ende des Zuleitungsabschnitts 18 der Konverterspule 9 ist ein Steckverbinderteil 11 vorgesehen, an den eine Leiterplatte 10 angeschlossen ist, wobei die Leiterplatte 10 und das Steckverbinderteil 11 innerhalb des Kanalabschnitts 16 integriert angeordnet sind. In den Kanalabschnitt 16 eingelassen sind zwei Kontaktstifte 6 vorgesehen, die an die Leiterplatte 10 angeschlossen sind. Die Kontaktstifte 6 sind jeweils in einem dazugehörigen Sackloch in dem Kanalabschnitt 16 angeordnet, wobei jeweils das freie Ende der Kontaktstifte 6 verbreitet mit einer planen Stirnfläche ausgestattet ausgebildet ist. Die Sacklöcher sind mit einer Umfangsstufe versehen ausgebildet, wobei um jeden Kontaktstift 6 herum angeordnet und an der der Stirnfläche der verbreiteten Stellen abgewandten Seite des jeweiligen Kontaktstifts 6 und an der Umfangsstufe des jeweiligen Sacklochs angreifend eine Druckfeder 7 vorgesehen ist. Der Kontaktstift 6 ist längs verschiebbar in seinem Sackloch und durch die Druckfeder 7 in Richtung zu dem Oberflächenabschnitt 5 hin nach außen vorgespannt, so dass, wenn die Konverterspuleneinheit 2 von dem Elektrolysefußbadgerät 1 abmontiert ist, die freien Enden der Kontaktstifte 6 an dem Oberflächenabschnitt 5 vorstehen.

In der Andockeinrichtung 4 sind zwei zu den Kontaktstiften 6 gehörende Gegenkontaktstifte 23 vorgesehen, die jeweils als Senkniet ausgebildet sind. Der Kopf eines jeden Senkniets ist plan mit dem Gegenoberflächenabschnitt 22. Die in das Elektrolysefußbadgerät 1 montierte Konverterspuleneinheit 2 ist mit ihrem Anschlussmittel 3 so an die Andockeinrichtung 4 angedockt, dass die Kontaktstifte 6 unter Vorspannung durch die Druckfedern 7 mit ihren verbreiteten Stirnseiten auf die mit dem Gegenoberflächenabschnitt 22 planen Oberflächen der Gegenkontaktstifte 23 gedrückt werden. Dadurch wird der elektrische Kontakt jeweils zwischen dem Gegenkontaktstift 6 und dem Gegenkontaktstift 23 hergestellt.

Die Gegenkontaktstifte 23 sind an eine Gegenleiterplatte 25 angeschlossen, wobei die Gegenleiterplatte 25 innerhalb der Fußbadschalenanordnung 26 integriert angeordnet ist.

An dem Oberflächenabschnitt 5 ist in den Kanalabschnitt 16 ein Magnet 8 eingelassen, und an dem Gegenoberflächenabschnitt 22 ist in die Andockeinrichtung 4 ein Gegenmagnet 24 eingelassen, wobei die Magnete 8, 24 zwischen den Kontaktstiften 6 bzw. den Gegenkontaktstiften 23 liegend angeordnet sind. Die Magnete 8, 24 haben an ihren zugewandten Oberflächen gegensinnige Pole, so dass sich beide Magnete 8, 24 durch magnetische Kraftwirkung gegenseitig anziehen. Somit wirkt auf das Anschlussmittel 3 eine Anziehungskraft, die die Konverterspuleneinheit 2 mit ihrem Anschlussmittel 3 an die Andockeinrichtung 4 angedockt festhält. Dadurch wird erreicht, dass die beiden Oberflächenabschnitte 5, 22 fest aneinander liegen, wobei der Kontaktstift 6 und der Gegenkontaktstift 23 flüssigkeitsdicht abgeschlossen sind.

Die Anziehungskraft zwischen den Magneten 8, 24 nimmt mit der dritten Potenz des Abstands der beiden Magnete 8, 24 zueinander ab. Liegt das Anschlussmittel 3 mit seinem Oberflächenabschnitt 5 an der Andockeinrichtung 4 mit seinem Gegenoberflächenabschnitt 22 an, so ist die Anhaftkraft zwischen dem Anschlussmittel 3 und der Andockeinrichtung 4 hoch, wodurch die mechanische Verbindung zwischen der Konverterspuleneinheit 2 und dem Elektrolysefußbadgerät 1 stabil ist. Wird die Konverterspuleneinheit 2 demontiert, beispielsweise durch Greifen der Öse 21 und Anheben der Konverterspuleneinheit 2 weg von dem Elektrolysefußbadgerät 1, so nimmt die Anziehungskraft zwischen den Magneten 8, 24 mit zunehmendem Abstand der beiden Magnete 8, 24 zueinander stark ab, so dass die Konverterspuleneinheit 2 leicht von dem Elektrolysefußbadgerät 1 montiert und demontiert werden kann.

Der Gegenoberflächenabschnitt 22 weist einen Vorsprung auf, der in eine dazugehörige Delle an dem Oberflächenabschnitt 5 formschlüssig eingreift. Dadurch ist ein seitliches Verrutschen der Konverterspuleneinheit 2 an dem Oberflächenabschnitt 5 bezogen auf den Gegenoberflächenabschnitt 22 unterbunden, wodurch eine mechanisch stabile Verbindung zwischen dem Anschlussmittel 3 und der Andockeinrichtung 4 erzielt wird. Ferner kann der Vorsprung und die Delle als Positionierhilfe zum Positionieren der Konverterspuleneinheit 2 mit ihrem Anschlussmittel 3 auf der Andockeinrichtung 4 des Elektrolysefußbadgeräts 1 genommen werden, da das Eingreifen des Vorsprungs in die Delle die korrekte Positionierung der Konverterspuleneinheit 2 signalisiert.

### Bezugszeichenliste

- 1: Elektrolysefußbadgerät
- 2: Konverterspuleneinheit
- 3: Anschlussmittel
- 4: Andockeinrichtung
- 5: Oberflächenabschnitt
- 6: Kontaktstift
- 7: Druckfeder
- 8: Magnet
- 9: Konverterspule
- 10: Leiterplatte
- 11: Steckverbinderteil
- 12: Trägerkörper
- 13: Hantelabschnitt
- 14: Stange
- 15: Teller
- 16: Kanalabschnitt
- 17: Windungsabschnitt
- 18: Zuleitungsabschnitt
- 19: Freies Ende des Kanalabschnitts
- 20: Schenkel des Kanalabschnitts
- 21: Öse
- 22: Gegenoberflächenabschnitt
- 23: Gegenkontaktstift
- 24: Gegenmagnet
- 25: Gegenleiterplatte
- 26: Fußbadschalenanordnung
- 27: Normfüllhöhe der Fußbadschalenanordnung

## Patentansprüche

1. Konverterspuleneinheit (2) für ein Elektrolysefußbadgerät (1), mit einem integrierten Anschlussmittel (3), mittels dessen die Konverterspuleneinheit (2) an eine Andockeinrichtung (4) andockbar ist.

2. Konverterspuleneinheit gemäß Anspruch 1, wobei das Anschlussmittel (3) einen Oberflächenabschnitt (5) aufweist, der stumpf an die Andockeinrichtung (4) zur Herstellung eines elektrischen Kontakts an dem Oberflächenabschnitt (5) anlegbar ist.

3. Konverterspuleneinheit gemäß Anspruch 2, wobei der Oberflächenabschnitt (5) Vertiefungen aufweist.

4. Konverterspuleneinheit gemäß Anspruch 2 oder 3, wobei das Anschlussmittel (3) wenigstens eine in das Anschlussmittel (3) eingelassenen und an dem Oberflächenabschnitt (5) von außen zugänglichen Kontaktstift (6) aufweist, der von einem Gegenkontaktelement der Andockeinrichtung (4) kontaktierbar ist.

5. Konverterspuleneinheit gemäß Anspruch 4, wobei der Kontaktstift (6) von dem Oberflächenabschnitt (5) vorsteht.

6. Konverterspuleneinheit gemäß Anspruch 4 oder 5, wobei der Kontaktstift (6) in dem Anschlussmittel (3) längs verschiebbar ist, wodurch der Kontaktstift (6) mit dem Gegenkontaktelement in Eingriff bringbar ist.

7. Konverterspuleneinheit gemäß einem der Ansprüche 4 bis 6, wobei das dem Oberflächenabschnitt (5) zugewandte Ende des Kontaktstifts eine konvexe Struktur aufweist, die mit einem dazugehörig entsprechend geformten Abschnitt des Gegenkontaktelements formschlüssig zusammenwirken kann.

8. Konverterspuleneinheit gemäß Anspruch 6 oder 7, wobei das Anschlussmittel (3) ein Vorspannmittel aufweist, das den Kontaktstift (6) in Richtung aus dem Anschlussmittel (3) heraus vorspannt.

9. Konverterspuleneinheit gemäß Anspruch 8, wobei das Vorspannmittel eine Druckfeder (7) ist.

10. Konverterspuleneinheit gemäß einem der Ansprüche 1 bis 9, wobei das Anschlussmittel (3) ein Haltemittel zum Halten des an die Andockeinrichtung (4) angedockten Anschlussmittels (3) in Position bezogen auf die Andockeinrichtung (4) aufweist.

11. Konverterspuleneinheit gemäß Anspruch 10, wobei das Haltemittel ein an dem Oberflächenabschnitt (5) in das Anschlussmittel (3) eingelassener Magnet (8) ist, der durch magnetische Kraftwirkung mit der Andockeinrichtung (4) zusammenwirkt, so dass an dem Oberflächenabschnitt (5) das Anschlussmittel (3) an der Andockeinrichtung (4) anhaftet.

12. Konverterspuleneinheit gemäß Anspruch 11, wobei der Magnet (8) ein Permanentmagnet ist.

13. Konverterspuleneinheit gemäß einem der Ansprüche 1 bis 12, wobei die Konverterspuleneinheit (2) eine Konverterspule (9) und das Anschlussmittel (3) eine Verbindungseinrichtung aufweist, mittels der via den Kontaktstift (6) die Konverterspule (9) mit der Andockeinrichtung (4) in elektrische Verbindung bringbar ist.

14. Konverterspuleneinheit gemäß Anspruch 13, wobei die Verbindungseinrichtung eine eine Logik aufweisende Leiterplatte (10) aufweist, an die der Kontaktstift (6) angebaut und die Konverterspule (9) angeschlossen ist.

15. Konverterspuleneinheit gemäß Anspruch 14, wobei die Verbindungseinrichtung ein Steckverbinderteil (11) aufweist, mittels dessen die Konverterspule (9) an die Leiterplatte (10) angeschlossen ist.

16. Konverterspuleneinheit gemäß einem der Ansprüche 13 bis 15, wobei die Konverterspuleneinheit (2) einen die Konverterspule (9) tragenden und das Anschlussmittel (3) integrierenden Trägerkörper (12) aufweist.

17. Konverterspuleneinheit gemäß Anspruch 16, wobei der Trägerkörper (12) einen Hantelabschnitt (13) mit einer Stange (14) und jeweils an deren Enden angeordneten Tellern (15) und einen hohlen Kanalabschnitt (16) aufweist, der an einem der Teller (15) beginnend von diesem sich weg erstreckt, und die Konverterspule (9) einen Windungsabschnitt (17) und einen Zuleitungsabschnitt (18) aufweist, wobei der Windungsabschnitt (17) um den Hantelabschnitt (13) angeordnet von diesem getragen und der Zuleitungsabschnitt (18) innerhalb des Kanalabschnitts angeordnet ist, wobei das Anschlussmittel (3) am freien Ende (19) des Kanalabschnitts (16) angesiedelt ist, so dass die Konverterspule (9) an dem Zuleitungsabschnitt (18) mit der Andockeinrichtung (4) in eine elektrische Verbindung bringbar ist.

18. Konverterspuleneinheit gemäß Anspruch 17, wobei der Kanalabschnitt (16) L-förmig geformt ist, wobei der eine Schenkel (20) radial nach außen von dem Hantelabschnitt (13) aus gesehen geführt ist und der andere Schenkel (28) axial von dem Hantelabschnitt (13) aus gesehen von der Stange (14) sich weg erstreckt.

19. Konverterspuleneinheit gemäß Anspruch 18, wobei der Oberflächenabschnitt (5) am außen liegenden Schenkel (28) des Kanalabschnitts (16) an der der Längsachse des Hantelabschnitts (13) zugewandten Seite angesiedelt ist und an der dem Oberflächenabschnitt (5) abgewandten Seite des Kanalabschnitts (16) eine Öse (21) als Griff angebracht ist.

20. Konverterspuleneinheit gemäß einem der Ansprüche 17 bis 19, wobei der Trägerkörper (12) einstückig geformt ist.

21. Konverterspuleneinheit gemäß Anspruch 20, wobei der Trägerkörper (12) aus einem spritzgussfähigen Kunststoff hergestellt ist.

22. Konverterspuleneinheit gemäß Anspruch 21, wobei die erste und/oder die letzte Windung des Windungsabschnitts (17) in dem jeweils benachbarten Teller (15) vergossen sind, so dass die Konverterspule (9) auf den Hantelabschnitt (13) gehalten ist.

23. Konverterspuleneinheit gemäß Anspruch 21 oder 22, wobei die erste und/oder die letzte Windung des Windungsabschnitts (17) von Vorsprüngen an dem jeweils benachbarten Teller (15) festgelegt sind, so dass die Konverterspule (9) auf dem Hantelabschnitt (13) gehalten ist.

24. Elektrolysefußbadgerät für eine Konverterspuleneinheit gemäß einem der Ansprüche 1 bis 23, mit einer Andockeinrichtung (4) an die die Konverterspuleneinheit (2) mittels eines Anschlussmittels (3) andockbar ist.

25. Elektrolysefußbadgerät gemäß Anspruch 24, wobei die Andockeinrichtung (4) einen Gegenoberflächenabschnitt (22) aufweist, an dem stumpf das Anschlussmittel (3) zur Herstellung eines elektrischen Kontakts an dem Gegenoberflächenabschnitt (22) anlegbar ist.

26. Elektrolysefußbadgerät gemäß Anspruch 25, wobei der Gegenoberflächenabschnitt (22) plan ist.

27. Elektrolysefußbadgerät gemäß Anspruch 25 oder 26, wobei die Andockeinrichtung (4) wenigstens einen in die Andockeinrichtung (4) eingelassenen und an dem Gegenoberflächenabschnitt (22) von außen zugänglichen Gegenkontaktstift (23) aufweist, der von einem Kontaktelement des Anschlussmittels (3) kontaktierbar ist.

28. Elektrolysefußbadgerät gemäß Anspruch 27, wobei der Gegenkontaktstift (23) in der Andockeinrichtung (4) versenkt ist.

29. Elektrolysefußbadgerät gemäß Anspruch 28, wobei der Gegenkontaktstift (23) als Senkniet ausgeführt ist.

30. Elektrolysefußbadgerät gemäß Anspruch 28 oder 29, wobei das dem Gegenoberflächenabschnitt (22) zugewandte Ende des Gegenkontaktstifts (23) eine konkave Struktur aufweist, die mit einem dazugehörig entsprechend geformten Abschnitt des Kontaktelements formschlüssig zusammenwirken kann.

31. Elektrolysefußbadgerät gemäß einem der Ansprüche 28 bis 30, wobei die Andockeinrichtung ein Gegenhaltemittel zum Halten des an die Andockeinrichtung (4) angedockten Anschlussmittels (3) in Position bezogen auf die Andockeinrichtung (4) aufweist.

32. Elektrolysefußbadgerät gemäß Anspruch 31, wobei das Gegenhaltemittel ein an dem Gegenoberflächenabschnitt (22) in die Andockeinrichtung (4) eingelassener Gegenmagnet (24) ist, der durch magnetische Kraftwirkung mit dem Anschlussmittel (3) zusammenwirkt, so dass an dem Gegenoberflächenabschnitt (22) das Anschlussmittel (3) an der Andockeinrichtung (4) anhaftet.

33. Elektrolysefußbadgerät gemäß Anspruch 32, wobei der Gegenmagnet (24) ein Permanentmagnet ist.

34. Elektrolysefußbadgerät gemäß Anspruch 33, wobei die Andockeinrichtung (4) eine eine Logik aufweisende Gegenleiterplatte (25) aufweist, an der der Gegenkontaktstift (23) angebaut ist.

35. Elektrolysefußbadgerät gemäß einem der Ansprüche 24 bis 34, wobei das Elektrolysefußbadgerät (1) eine Fußbadschalenanordnung (26) aufweist, in die die Andockeinrichtung (4) integriert ist.

36. Elektrolysefußbadgerät gemäß Anspruch 35, wobei der Gegenoberflächenabschnitt (22) als Teil der Oberfläche der Fußbadschalenanordnung (26) an einem Ort angesiedelt ist, der oberhalb der Normfüllhöhe (27) der Fußbadschalenanordnung (26) ist.

37. Elektrolysefußbadgerät gemäß Anspruch 35, wobei der Gegenoberflächenabschnitt (22) außerhalb der Fußbadschalenanordnung (26) angesiedelt ist.

38. Elektrolysefußbadgerät gemäß einem der Ansprüche 24 bis 37, wobei zwei Kontakte zur Energieübertragung und zwei Kontakte zur Datenübertragung vorgesehen sind.

39. Elektrolysefußbadgerät gemäß einem der Ansprüche 24 bis 38, wobei der Gegenoberfächenabschnitt (22) eine erhabene Stelle aufweist, die mit einer dazugehörigen zurückversetzten Stelle an dem Oberflächenabschnitt formschlüssig zusammenwirken kann.
